# EUROPÄISCHE PATENTSCHRIFT

(11) **EP 4 035 614 B1**
(45) Veröffentlichungstag und Bekanntmachung des Hinweises auf die Patenterteilung: **01.01.2025**
(21) Anmeldenummer: 21211052.2
(22) Anmeldetag: 29.11.2021
(51) Int. Cl.: A61B 17/32, A61B 17/00, H01H 25/00

(54) **MEDIZINISCHES INSTRUMENT MIT VERBESSERTER HAPTISCHER HANDHABUNG**
MEDICAL INSTRUMENT WITH IMPROVED HAPTIC HANDLING
INSTRUMENT MÉDICAL À MANIPULATION HAPTIQUE AMÉLIORÉE

(30) Priorität: 28.01.2021 DE 102021101903
(43) Veröffentlichungstag der Anmeldung: 03.08.2022
(73) Patentinhaber: Eberle GmbH & Co. KG, 75449 Wurmberg (DE)
(72) Erfinder: LÖFFLER, Heiko, 75449 Wurmberg (DE)
(74) Vertreter: Hoefer & Partner Patentanwälte mbB

(56) Entgegenhaltungen:
- EP-A1- 2 564 795
- WO-A1-2006/125558
- FR-A1- 2 368 790
- JP-A- 2004 179 047
- US-A1- 2008 287 925
- US-B1- 6 500 169

## Beschreibung

Die vorliegende Erfindung betrifft ein medizinisches Instrument, insbesondere für arthroskopische Eingriffe in Knie oder Hüfte oder Sprunggelenk, welches eine vereinfachte Handhabung ermöglicht und verbesserte haptische Eigenschaften aufweist.

Medizinische Instrumente für arthroskopische Eingriffe sind aus dem Stand der Technik in unterschiedlichen Ausgestaltungsformen bekannt. Das medizinische Instrument umfasst üblicherweise ein Handstück und einen Schneidenhalter. Die Handstücke sind dabei mit einem Antrieb verbunden oder weisen einen Antrieb auf, so dass Schneideninstrumente im Schneidenhalter rotativ angetrieben werden können, um Gewebe oder anderes biologisches Material insbesondere aus einem Gelenkbereich zu entfernen. Üblicherweise weisen derartige medizinische Instrumente ferner noch eine Absaugeinrichtung zum Absaugen von abgetrenntem Material auf, welche durch das medizinische Instrument hindurchgeführt wird. Weiterhin ist der Schneidenhalter lösbar am Handstück angebracht, damit eine entsprechende Reinigung der Teile des medizinischen Instruments möglich ist. Hierbei sollte das Lösen und Verbinden von Handstück und Schneidenhalter möglichst schnell, einfach und sicher sein. Eine derartige Verbindungsanordnung ist hierbei beispielsweise aus der EP 1 790 292 A1 bekannt. Diese Verbindungsanordnung umfasst jedoch relativ viele Teile und ist nach einem Gebrauch relativ aufwendig zu reinigen. Weiterhin muss für einen Chirurg auch eine optimale Handhabung während einer Operation möglich sein. Üblicherweise hat der Chirurg bei der Operation seinen Blick nicht auf seine Hand gerichtet, in welcher er das medizinische Instrument hält. Zur Bedienung des medizinischen Instruments sind am Handstück üblicherweise mehrere einzelne Tasten angeordnet, welche der Chirurg mit einem Finger, häufig mit dem Daumen, betätigen muss, um einen Linkslauf oder einen Rechtslauf oder eine Absaugung mit dem medizinischen Instrument durchzuführen. Eine Fehlbedienung durch das Drücken eines falschen Knopfes könnte bei der Operation hier zu unerwünschten Ergebnissen führen. Da die Handstücke nach einer Operation wiederverwendet werden, muss auch eine rückstandsfreie Reinigung der Handstücke sichergestellt werden. Aus der WO 2006/12558 A1 ist ein Instrument mit einer Schaltwippe bekannt, bei der durch Wippen um eine Wippachse ein erster oder zweiter Kontakt betätigt werden kann oder durch Verschieben der Wippachse beide Kontakte gleichzeitig betätigt werden können. Die FR 2 368 790 A1 offenbart ferner einen Schalter, bei dem drei Sensoren separat betätigt werden können. Ferner ist aus der JP 2004-179 047 A ein Knopfschalter bekannt, welcher durch Verschwenken eines Knopfhalters, der mit einem Knopf verbunden ist, einen von drei Kontakten kontaktieren kann. Die EP 2 564 795 A1, US 2008/287925 A1 und US 6 500 169 B1 beschreiben medizinische Geräte mit einem Handstück, welches drei Taster aufweist. Die Taster sind unabhängig voneinander in Reihe an einem distalen Ende angeordnet.

Es ist daher Aufgabe der vorliegenden Erfindung, ein medizinisches Instrument bereitzustellen, welches bei einfachem Aufbau und einfacher, kostengünstiger Herstellbarkeit eine sichere und einfache Bedienung ermöglicht sowie eine besonders einfache und schnelle Reinigung möglich macht.

Diese Aufgabe wird durch ein medizinisches Instrument mit den Merkmalen des Anspruchs 1 gelöst. Die Unteransprüche zeigen bevorzugte Weiterbildungen der Erfindung.

Das medizinische Instrument für arthroskopische Eingriffe mit den Merkmalen des Anspruchs 1 weist demgegenüber den Vorteil auf, dass eine haptische Wahrnehmung für einen Nutzer des medizinischen Instruments deutlich verbessert ist. Insbesondere ist das erfindungsgemäße medizinische Instrument geeignet, nur mit einem Finger, vorzugsweise mit einem Daumen des Nutzers betätigt zu werden. Dabei gibt das erfindungsgemäße Instrument dem Nutzer eine optimale Rückmeldung, wenn eine Taste des Handstücks betätigt wird. Dies wird erfindungsgemäß dadurch erreicht, dass eine Tastenanordnung zur Betätigung des medizinischen Instruments vorgesehen ist, welche nahe einem distalen Ende des Handstücks, an welchem ein Schneidenhalter am Handstück angeordnet ist, vorgesehen ist. Dabei umfasst die Tastenanordnung drei Tasten, jedoch nur zwei Tastkontakte. Hierbei ist eine erste Taste eingerichtet, einen ersten Tastkontakt zu betätigen. Eine zweite Taste ist eingerichtet, einen zweiten Tastkontakt zu betätigen. Eine dritte Taste ist dabei eingerichtet, gleichzeitig den ersten und zweiten Tastkontakt zu betätigen. Dadurch weist das medizinische Instrument einen deutlich vereinfachten Aufbau auf, da nicht für jede Taste jeweils ein Tastkontakt vorhanden ist, sondern zwei Tastkontakte in unterschiedlicher Weise mittels dreier Tasten betätigt werden können. Weiterhin kann durch die Anordnung der Tastenanordnung nahe dem distalen Ende des Handstücks, an welchem der Schneidenhalter, welcher ein äußeres Hohlrohr und ein inneres hohles Schneidenrohr mit Schneiden an einem distalen Ende des Innenrohrs aufweist, erreicht werden, so dass das medizinische Instrument sehr gut in der Hand liegt. Dabei ist das medizinische Instrument für einen Nutzer sehr gut in der Hand ausbalanciert, so dass insbesondere eine Betätigung der Tastenanordnung mittels eines Daumens des Nutzers einfach und sicher möglich ist.

Weiter sind die Tasten an einer gemeinsamen, starren Tastenplatte angeordnet. Dies ermöglicht neben einer besonders einfachen und kostengünstigen Herstellbarkeit auch reinigungstechnisch große Vorteile, da weniger Spalte und Ritzen zwischen den Tasten vorhanden sind. Die starre Tastenplatte hat den weiteren Vorteil, das auch auf einfache Weise sichergestellt werden kann, dass mittels der dritten Taste beide Tastkontakte gleichzeitig betätigt werden können. Die starre Tastenplatte ist dabei vorzugsweise aus Metall, insbesondere Edelstahl, hergestellt.

Ferner ist die starre Tastenplatte lose auf dem ersten und zweiten Tastkontakt angeordnet. Die starre Tastenplatte ist dabei an ihren axialen Längsseiten an einem Deckel oder einer Abdeckung oder dergleichen gehalten, wobei insbesondere ein vorspringender Bereich der Tastenplatte hinter eine Hinterschneidung am Deckel eingreift.

Besonders bevorzugt sind die drei Tasten der Tastenanordnung in Axialrichtung des Handstücks in Reihe angeordnet. Dabei ist die dritte Taste, welche eingerichtet ist, gleichzeitig den ersten und zweiten Tastkontakt zu betätigen, zwischen der ersten und zweiten Taste angeordnet. Dadurch sind die drei Tasten in sehr kompakter Weise am Handstück positioniert, so dass eine Bedienung sehr einfach ist. Die Tastenanordnung ist besonders bevorzugt an einer Oberseite des Handstücks angeordnet. Dadurch kann das Handstücks derart in der Hand gehalten werden, dass der Daumen an der Oberseite des Handstücks im Bereich der Tastenanordnung positioniert ist und die Unterseite des Handstücks auf den restlichen Fingern des Nutzers aufliegt und sicher gehalten werden kann.

Besonders bevorzugt weist die starre Tastenplatte speziell ausgestaltete Tasten auf, welche an einer Oberseite der starren Tastenplatte ausgebildet sind. Die starre Tastenplatte weist an der ersten Taste an einem distalen Ende der Tastenplatte einen ersten erhöhten Bereich auf. An der zweiten Taste weist die Tastenplatte an einem proximalen Ende einen zweiten erhöhten Bereich auf. Weiterhin weist die dritte Taste, die in der Mitte zwischen der ersten und zweiten Taste angeordnet ist, einen dritten erhöhten Bereich auf. Durch das Vorsehen der erhöhten Bereiche können die drei Tasten der Tastenplatte einfach und sicher gefunden werden und entsprechend sicher betätigt werden.

Besonders bevorzugt verjüngt sich dabei die erste Taste in Richtung zur dritten Taste und die zweite Taste verjüngt sich ebenfalls in Richtung der dritten Taste. Dadurch weist die Tastenplatte an ihren beiden Enden die erhöhten Bereiche auf und im Bereich der dritten Taste in der Mitte ist der dritte erhöhte Bereich vorgesehen. Ein Nutzer kann somit ausgehend vom erhöhten Bereich der ersten Taste erfühlen, dass sich die erste Taste in Richtung zur Mitte verjüngt, dann die nächste Erhöhung die dritte Taste mit dem dritten erhöhten Bereich ist und bei weiterem Bewegen des Daumens in Richtung zur zweiten Taste kann der Nutzer zuerst das Ende der dritten Taste erfühlen und dann das langsame Ansteigen bis zum zweiten erhöhten Bereich der zweiten Taste erfühlen. Dadurch kann eine Betätigung der drei Tasten ohne Hinsehen auf sichere und einfache Weise sichergestellt werden.

Vorzugsweise ist im unbetätigten Zustand ein Abstand des ersten erhöhten Bereichs, des zweiten erhöhten Bereichs und des dritten erhöhten Bereichs von einer Mittelachse des medizinischen Instruments gleich.

Gemäß einer weiteren bevorzugten Ausgestaltung der Erfindung umfasst das medizinische Instrument ferner eine Steuerplatine, welche in die Tastenanordnung integriert ist. Die Steuerplatine ist vorzugsweise unterhalb der starren Tastenplatte angeordnet. Der erste und zweite Tastkontakt ist dabei zwischen der starren Tastenplatte und der Steuerplatine angeordnet. Vorzugsweise sind Rückstellelemente zwischen den Tastkontakten und der Steuerplatine angeordnet. Die Steuerplatine ist vorzugsweise derart ausgebildet, dass auf einer Oberfläche der Steuerplatine Kontaktbereiche vorgesehen sind, welche durch Betätigung der Tastenplatte kontaktiert werden können, um entsprechende Steuerungsbefehle, insbesondere einen rotierenden Antrieb des inneren Schneidenrohrs zu ermöglichen.

Besonders bevorzugt umfasst die Tastenanordnung ein erstes und zweites Rückstellelement. Die beiden Rückstellelemente sind dabei am ersten bzw. zweiten Tastkontakt angeordnet und ermöglichen eine Rückstellung der Tastenanordnung in eine unbetätigte Ausgangsstellung. Die Rückstellelemente sind vorzugsweise unterhalb der Tastkontakte angeordnet. Die Rückstellelemente sind dabei besonders bevorzugt Schnappscheiben, vorzugsweise aus Federstahl.

Das erste und zweite Rückstellelement ist vorzugsweise jeweils eine federnde Schnappscheibe, welche bei einer Verformung ein akustisches Signal, beispielsweise ein Knacken, erzeugt, so dass ein Nutzer auch eine akustische Rückmeldung betreffend ein Drücken einer Taste erhält. Die Schnappscheibe erzeugt ferner auch bei ihrer Rückstellung in die Ausgangsposition wieder ein akustisches Signal.

Die dritte Taste an der starren Tastenplatte ist vorzugsweise als kleine zylindrische Erhebung auf der Tastenplatte ausgebildet. Die dritte Taste ist vorzugsweise knopfartig ausgebildet.

Weiter bevorzugt weist die starre Tastenplatte in Axialrichtung im Bereich der dritten Taste eine symmetrische Einschnürung auf. Hierbei ist eine Breite der starren Tastenplatte im Bereich der dritten Taste kleiner als im Bereich der ersten und zweiten Taste. Dadurch wird eine Erkennung für einen Nutzer, an welcher Stelle er sich gerade mit seinem Finger befindet, weiter vereinfacht.

Weiterhin weist das medizinische Instrument den Vorteil auf, dass ein schnelles und einfaches Lösen eines Schneidenhalters von einem Handstück sowie ein schnelles Verbinden des Schneidenhalters mit dem Handstück möglich ist. Weiterhin können sowohl das Handstück als auch der Schneidenhalter sehr einfach und gründlich in kurzer Zeit gereinigt werden. Dies wird dadurch erreicht, dass das medizinische Instrument eine Verbindungsanordnung zum lösbaren Verbinden des Handstücks mit dem Schneidenhalter aufweist. Der Schneidenhalter umfasst dabei ein Außenrohr und ein Innenrohr mit Schneiden am distalen Ende. Die Verbindungsanordnung weist zwei sich in Axialrichtung des medizinischen Instruments erstreckende Arme auf, die am Schneidenhalter angeordnet sind. Die Arme liegen sich einander gegenüber und weisen an ihren freien Enden ein Rastelement auf. Ferner weisen die Arme jeweils einen Drückbereich zur Betätigung und einen elastischen Bereich auf. Das Handstück weist an einem Innenumfang eine Aufnahme mit einer Hinterschneidung für die Rastelemente auf. Darüber hinaus weist das Handstück an einem distalen Endbereich zwei Ausnehmungen auf, welche zur teilweisen Aufnahme der Arme dienen. Die Rastelemente und die Hinterschneidung stellen eine axiale Sicherung des Schneidenhalters am Handstück bereit. Die zwei Ausnehmungen am distalen Endbereich des Handstücks und die in den Ausnehmungen angeordneten Bereiche der Arme stellen eine Verdrehsicherung bereit. Somit weist die erfindungsgemäße Verbindungsanordnung zwischen dem Handstück und dem Schneidenhalter neben der axialen Sicherung gleichzeitig auch eine Verdrehsicherung auf. Dabei ist eine Teileanzahl der Verbindungsanordnung sehr klein. Weiterhin können die Teile der Verbindungsanordnung schnell und einfach gereinigt werden. Dadurch ist die Verbindungsanordnung insbesondere geeignet, bei mehrfach verwendbaren Schneidenhaltern eingesetzt zu werden. Da die Verbindungsanordnung auch relativ kostengünstig herstellbar ist, beispielsweise aus einem Kunststoff, ist die Verbindungsanordnung jedoch auch geeignet, bei Einmal-Instrumenten verwendet zu werden.

Vorzugsweise stehen die Drückbereiche von den Armen radial nach außen vor. Die Drückbereiche stehen dabei besonders bevorzugt von einem Außenumfang des Handstücks im Bereich der Verbindungsanordnung nach außen vor, so dass eine einfache Betätigung, insbesondere zum Lösen des Schneidenhalters vom Handstück, ermöglicht wird.

Weiter bevorzugt weisen die Rastelemente an den Armen eine Rampe auf. Die Rampe weist vorzugsweise einen Winkel von ≤ 45° auf. Dadurch kann eine besonders einfache Verbindung durch eine in Axialrichtung erfolgte Relativverschiebung zwischen dem Schneidenhalter und dem Handstück erfolgen, so dass der Schneidenhalter einfach auf das Handstück aufgesetzt werden kann und in Axialrichtung bewegt werden kann. Die Arme gleiten dann an den Rampen in einem Innenbereich des Handstücks und rasten federnd hinter der Hinterschneidung am Handstück ein.

Weiter bevorzugt sind die Arme einstückig mit einem Ring ausgebildet, wobei sich der elastische Bereich der Arme an einem Bereich zwischen dem Ring und den Drückbereichen befindet. Dadurch kann eine Befestigung der Verbindungsanordnung mittels des Rings am Schneidenhalter erfolgen und die Arme müssen lediglich die Funktionen der Verdrehsicherung und der axialen Sicherung übernehmen.

Der Ring, an welchem die Arme befestigt sind, ist vorzugsweise lösbar mittels einer Clipsverbindung mit dem Schneidenhalter verbunden. Dadurch ist es auch möglich, dass der Ring mitsamt den Armen vom Schneidenhalter gelöst werden kann und separat eine Reinigung von Schneidenhalter und diesem Teil der Verbindungsanordnung mit Ring und Armen möglich ist.

Die Ausnehmungen im Handstück zur Aufnahme von Teilbereichen der Arme sind vorzugsweise U-förmig ausgebildet. Dabei weisen die Arme entsprechend den Ausnehmungen U-förmige Bereiche, insbesondere an den Drückbereichen der Arme, auf.

Weiter bevorzugt ist die Tastenordnung in Umfangsrichtung zwischen den Ausnehmungen für die Aufnahme der Arme der Verbindungsanordnung angeordnet. Dadurch kann ein besonders kompakter Aufbau des medizinischen Instruments, insbesondere in Axialrichtung ermöglicht werden.

Durch diesen Aufbau der Tastenanordnung kann eine große bauliche Vereinfachung der Tastenanordnung ermöglicht werden. Dabei können zwei Tastkontakte durch drei Tasten unterschiedlich betätigt werden, so dass drei Betriebszustände des medizinischen Instruments mit nur zwei Tastkontakten möglich sind. Beispielsweise ist die erste Taste für einen Rechtslauf des medizinischen Instruments eingerichtet, die zweite Taste für einen Linkslauf des medizinischen Instruments eingerichtet und die dritte Taste für eine oszillierende Bewegung des medizinischen Instruments in Links-Rechts-Richtung eingerichtet. Die starre Tastenplatte liegt dabei über dem ersten und zweiten Tastkontakt. Die starre Tastenplatte ist vorzugsweise um eine Kippachse, die senkrecht zur Mittelachse X-X des medizinischen Instruments ist, kippbar.

Weiter bevorzugt umfasst das medizinische Instrument ferner eine Absaugungsregulierung, welche am Handstück in Reihe mit der Tastenanordnung angeordnet ist. Dadurch kann das medizinische Instrument in einem Einhand-Betrieb verwendet werden, wobei gleichzeitig das Schneideninstrument in entsprechender Richtung betätigt werden kann und eine Absaugung von abgetrenntem Gewebe reguliert werden kann.

Das medizinische Instrument wird vorzugsweise bei Gelenk-Operationen oder Meniskus-Operationen oder anderen endoskopischen Eingriffen verwendet.

Nachfolgend wird ein bevorzugtes Ausführungsbeispiel der Erfindung unter Bezugnahme auf die begleitende Zeichnung im Detail beschrieben. In der Zeichnung ist:
- Fig. 1: eine schematische, perspektivische Ansicht eines medizinischen Instruments gemäß einem bevorzugten Ausführungsbeispiel der Erfindung,
- Fig. 2: eine schematische, perspektivische Explosionsdarstellung einer Tastenanordnung des medizinischen Instruments von Fig. 1,
- Fig. 3: eine schematische Draufsicht der Tastenanordnung,
- Fig. 4: eine schematische Schnittansischt entlang der Linie IV-IV von Fig. 3,
- Fig. 5: eine schematische Längsschnittansicht durch die Tastenanordnung im unbetätigten Zustand,
- Fig. 6: eine schematische Längsschnittansicht der Tastenanordnung in einem betätigten Zustand,
- Fig. 7: eine schematische Schnittansicht durch ein Handstück des medizinischen Instruments von Fig. 1,
- Fig. 8: eine schematische Ansicht des Schneidenhalters mit einem Teil der Verbindungsanordnung zur Verbindung mit dem Handstück, und
- Fig. 9: eine schematische, perspektivische Ansicht des am Schneidenhalter angeordneten Teils der Verbindungsanordnung.

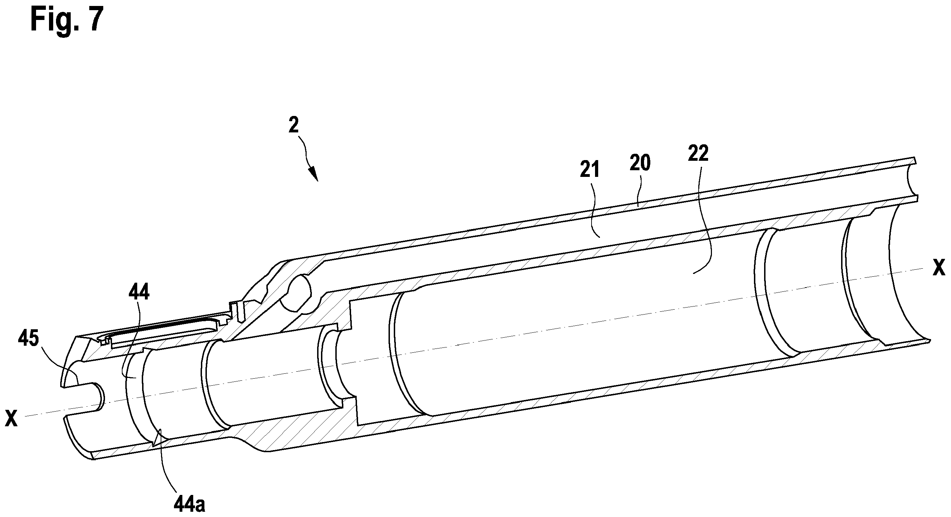

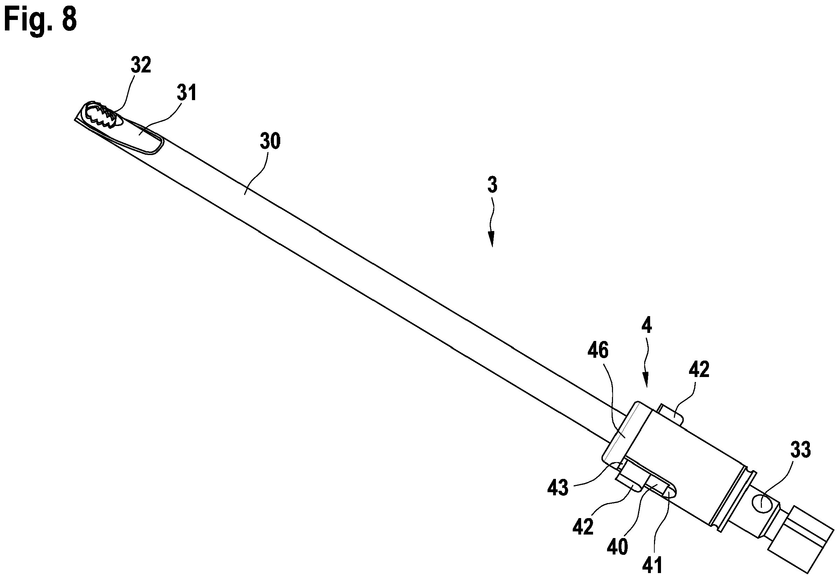

Nachfolgend wird unter Bezugnahme auf die Fig. 7 bis 9 ein medizinisches Instrument 1 für arthroskopische Eingriffe gemäß einem ersten bevorzugten Ausführungsbeispiel der Erfindung im Detail beschrieben.

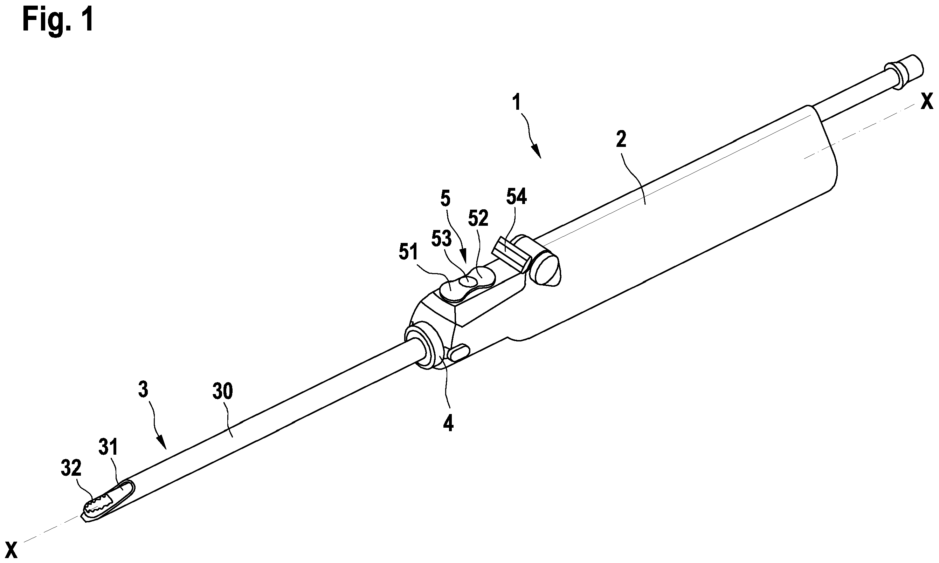

Wie aus Fig. 1 ersichtlich ist, umfasst das medizinische Instrument 1 ein Handstück2 und einen Schneidenhalter 3.

Im Handstück 2 ist beispielsweise im Hohlraum 22 ein Antrieb angeordnet oder alternativ ist das Handstück mit einem Antrieb verbindbar.

Der Schneidenhalter 3 umfasst ein äußeres Hohlrohr 30 und ein inneres Schneidenrohr 31 mit Schneiden 32 an einem distalen Ende.

Am Schneidenhalter 3 ist ferner ein Teil einer Verbindungsanordnung 4 angeordnet. Die Verbindungsanordnung 4 ist im Detail aus den Fig. 7 bis 9 ersichtlich.

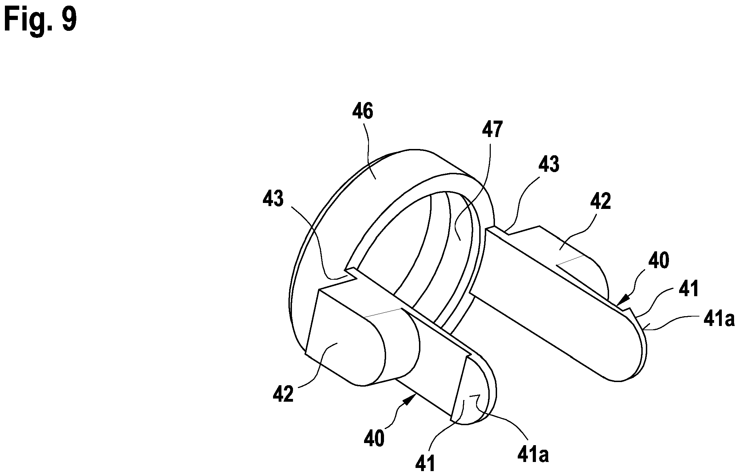

Wie insbesondere aus Fig. 9 ersichtlich ist, umfasst der Teil der Verbindungsanordnung 4, welcher am Schneidenhalter 3 angeordnet ist, einen Ring 46, von welchem zwei Arme 40 in Axialrichtung X-X des medizinischen Instruments vorstehen. Die beiden Arme 40 liegen sich am Ring 46 einander gegenüber. Der Ring 46 umfasst eine Clips-Verbindung 47, mit welcher der Ring an den Schneidenhalter 3 angeclipst werden kann. Dadurch ist der Ring 46 lösbar am Schneidenhalter 3 angeordnet.

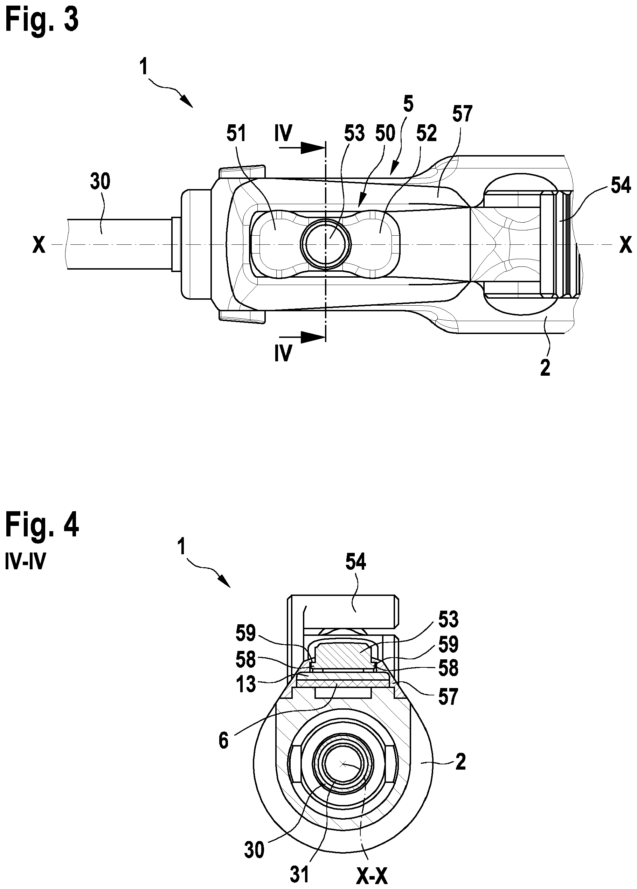

Die beiden Arme 40 sind gleich aufgebaut und weisen ein Rastelement 41 an einem distalen Ende, einen Drückbereich 42 zur Betätigung und einen elastischen Bereich 43 auf. Der elastische Bereich 43 ist dabei in Axialrichtung X-X zwischen dem Drückbereich und dem Ring 46 angeordnet. Der Drückbereich 42 ist Vollkörperbereich, welcher radial an den Armen 40 nach außen vorsteht. Wie insbesondere aus den Fig. 1 und 3 deutlich wird, stehen die Drückbereiche 43 somit seitlich vom medizinischen Instrument 1 vor. Dadurch ist ein einfaches Greifen und Betätigen der Drückbereiche zum Lösen des Schneidenhalters 3 vom Handstück 2 möglich.

Wie weiter aus Fig. 7 ersichtlich ist, umfasst die Verbindungsanordnung 4 im Handstück 2 in einem hohlen Handstückkörper 20 ferner eine Aufnahme 44 mit einer Hinterschneidung 44a. Die Hinterschneidung 44a verläuft als nutförmige Vertiefung entlang des Innenumfangs des hohlen Handstücks und kommt im montierten Zustand des Schneidenhalters 2 am Handstück 2 mit den Rastelementen 41 in Kontakt. Die Rastelemente 41 weisen dabei jeweils Rampen 41a auf, so dass durch einfaches axiales Aufschieben des Schneidenhalters 3 auf das Handstück 2 eine Verbindung hergestellt werden kann.

Dabei sind im Handstückkörper 20 ferner noch zwei U-förmige Ausnehmungen 45 ausgebildet. Die Ausnehmungen 45 dienen dabei zur Aufnahme der Drückbereiche 42. Dabei dienen die zwei Ausnehmungen 45 auch als Führungsbereich während des Aufschiebvorgangs des Schneidenhalters 3 auf das Handstück 2.

Im montierten Zustand des Schneidenhalters 3 am Handstück 2 sind somit die Druckbereiche 42 in den Ausnehmungen 45 aufgenommen und stehen radial nach außen über das Handstück 2 vor. Dadurch bilden die Drückbereiche 42 in Verbindung mit den Ausnehmungen 45 eine Verdrehsicherung des Schneidenhalters 3 gegenüber dem Handstück 2. Die Rastelemente 41, welche an der Hinterschneidung 44a eingerastet sind, bilden dabei eine axiale Sicherung des Schneidenhalters 3 am Handstück 2.

Somit kann eine verdrehsichere Verbindung zwischen dem Schneidenhalter 3 und dem Handstück 2 realisiert werden. Da die Verbindungsanordnung 4 auch vom Schneidenhalter 3 entfernbar ist, kann auch eine besonders einfache Reinigung der Teile des medizinischen Instruments ermöglicht werden.

Dabei ist die Verbindungsanordnung 4, die am Schneidenhalter 3 angeordnet ist, vorzugsweise ein Kunststoffteil. Durch das Vorstehen der Drückbereiche 42 von den Armen 40 kann auch eine einfache und sichere Betätigung durch einen Nutzer des medizinischen Instruments erfolgen.

Im Handstückkörper 20 ist ferner noch ein Absaugkanal 21 vorgesehen, über den abgesaugtes Gewebe, welches aus dem Schneidenhalter 3 aus einer Austrittsöffnung 33 austritt, abgeführt werden kann.

Am Handstück 2 ist ferner eine Tastenanordnung 5 zur Betätigung des medizinischen Instruments angeordnet. Die Tastenanordnung 5 umfasst eine erste Taste 51, eine zweite Taste 52 und eine dritte Taste 53. Wie aus Fig. 1 und 3 ersichtlich ist, sind die drei Tasten 51, 52, 53 in Axialrichtung X-X des medizinischen Instruments in Reihe angeordnet. Dabei ist die Tastenanordnung 5 an einem distalen Ende des Handstücks 2 nahe am Übergang zum Schneidenhalter 3 positioniert.

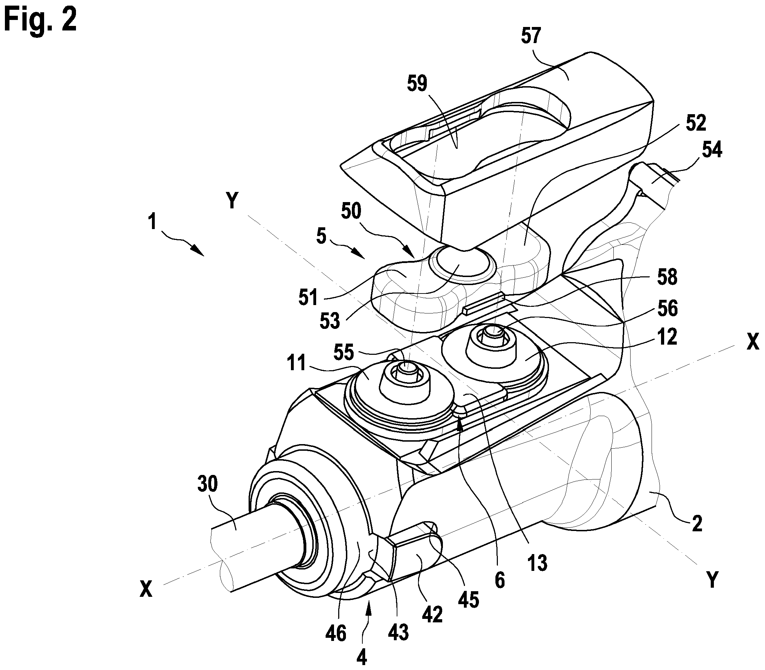

Die erste, zweite und dritte Taste 51, 52, 53 sind dabei auf einer starren Tastenplatte 50, vorzugsweise aus Edelstahl, integriert. Wie aus Fig. 2 und 3 ersichtlich ist, ist die Tastenplatte 50 länglich ausgebildet und liegt über einem ersten Tastkontakt 55 und einem zweiten Tastkontakt 56. Zwischen den beiden Tastkontakten 55, 56 befindet sich eine Kippachse Y-Y der Tastenanordnung. Die Kippachse Y-Y ist senkrecht zur Axialrichtung X-X des medizinischen Instruments 1. Die drei Tasten 51, 52, 53 sind somit Tastenbereiche an der starren Tastenplatte 50.

Die Tastenanordnung 5 umfasst ferner einen Deckel 57, welcher teilweise die Tastenplatte und insbesondere die Tastenkontakte 55, 56 vor äußeren Einflüssen schützt. Wie aus Fig. 2 ersichtlich ist, sind im Deckel 57 zwei seitliche Aussparungen 59 vorgesehen, welche in Axialrichtung X-X verlaufen. An der Tastenplatte 50 sind zwei ebenfalls in Axialrichtung X-X verlaufende seitliche Vorsprünge 58 vorgesehen, welche im montierten Zustand in die seitlichen Aussparungen 59 im Deckel 57 eingreifen. Dadurch ist die Tastenplatte 50 in Vertikalrichtung senkrecht zur Axialrichtung X-X und auch in Axialrichtung X-X gesichert.

Weiterhin ist unter dem ersten und zweiten Tastkontakt 55, 56 eine Steuerplatine 6 in die Tastenanordnung 5 integriert. Dadurch ist es möglich, dass durch Betätigung des ersten und/oder zweiten Tastkontakts 55, 56 direkt elektronische Steuerelemente auf der Steuerplatine 6 aktiviert werden können, um das medizinische Instrument 1 zu steuern.

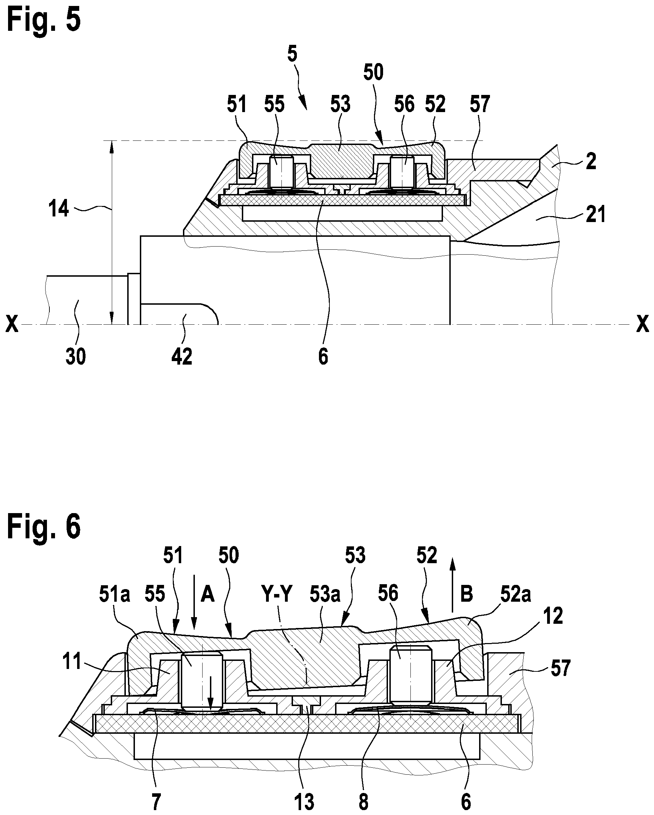

Die Tastenanordnung 5 umfasst somit nur genau die zwei Tastkontakte 55, 56. Der erste Tastkontakt 55 ist dabei unterhalb der ersten Taste 51 angeordnet und der zweite Tastkontakt 56 ist unterhalb der zweiten Taste 52 angeordnet. Somit kann durch Betätigung der ersten Taste 51 der erste Tastkontakt 55 aktiviert werden und durch Betätigung der zweiten Taste 52 der zweite Tastkontakt 56 aktiviert werden. Wie aus Fig. 1 und 5 ersichtlich ist, ist die dritte Taste 53 zwischen der ersten Taste 51 und der zweiten Taste 52 angeordnet. Die Tasteinrichtung 5 ist nun derart eingerichtet, dass durch Betätigung der dritten Taste 53 beide Tastkontakte 55, 56 gemeinsam mittels der starren Tastenplatte 50 betätigt werden.

Fig. 5 zeigt dabei den unbetätigten Zustand der Tastenanordnung 5. Die starre Tastenplatte 50 liegt dabei lose auf dem ersten und zweiten Tastkontakt 55, 56 auf. Wie insbesondere aus den Fig. 5 und 6 ersichtlich ist, ist zwischen der Steuerplatine 6 und dem ersten und zweiten Tastkontakt 55, 56 jeweils ein Rückstellelement angeordnet. Genauer ist unter dem ersten Tastkontakt 55 ein erstes Rückstellelement 7 angeordnet und unter dem zweiten Tastkontakt 56 ein zweites Rückstellelement 8. Die beiden Rückstellelemente 7, 8 sind als Schnappscheiben ausgebildet, welche bei einer Betätigung direkt auf die Steuerplatine 6 drücken und entsprechend einen Kontakt auf der Steuerplatine 6 schließen.

Fig. 6 zeigt dabei den Zustand, in welchem die erste Taste 51 betätigt ist. Dabei drückt ein Nutzer, wie durch den Pfeil A in Fig. 6 angedeutet, auf die erste Taste 51, wodurch der erste Tastkontakt in Richtung auf die Steuerplatine 6 bewegt wird und das erste Rückstellelement 7 verformt wird, um den Kontakt auf der Steuerplatine herzustellen. Da die Tastenplatte 50 starr ist, hebt sich, wie in Fig. 6 durch den Pfeil B angedeutet, die zweite Taste 52 in entgegengesetzter Richtung vom zweiten Tastkontakt 56 ab. Dabei schwenkt die starre Tastenplatte 50 um die Kippachse Y-Y.

Zum Schutz und zur Abdichtung der Steuerplatine 6 sind am ersten Tastkontakt 55 ein erstes Gehäuse 11 und am zweiten Tastkontakt 56 ein zweites Gehäuse 12 vorgesehen. Die beiden Gehäuse 11, 12 sind mittels einer Halteplatte 13 (vgl. insbesondere Fig. 2) auf dem Handstückkörper 20 gehalten. Das erste und zweite Gehäuse 11, 12 weisen dabei einen im Wesentlichen zylindrischen Führungsbereich auf, in welchem jeweils der erste und zweite Tastkontakt 55, 56, welche in diesem Ausführungsbeispiel zylindrische Bauteile sind, angeordnet sind. Hierbei ist eine nicht gezeigte Abdichtung zwischen den zylindrischen Tastkontakten 55, 56 und den zylinderförmigen Gehäuseteilen des ersten und zweiten Gehäuses 11, 12 ausgebildet. Somit kann die Steuerplatine 6 auf einfache Weise fluiddicht gegenüber der Umgebung geschützt sein, so dass auch bei einer Reinigung im Autoklav oder mittels anderer Reinigungstechniken keine Beschädigung der Tastenanordnung 5 und der Steuerplatine 6 auftritt.

Wie insbesondere aus Fig. 1 ersichtlich ist, ist die Tastenanordnung 50 an einer Oberseite des Handstücks 2 angeordnet. Dadurch kann ein Nutzer das Handstück beispielsweise derart in die Hand nehmen, dass mit dem Daumen die Tasten der Tastenanordnung 5 betätigt werden und das Handstück mit den restlichen Fingern in der Hand sicher gehalten werden kann.

Insbesondere ist eine deutlich verbesserte haptische Rückmeldung durch die Tastenanordnung 5 möglich. Wie insbesondere aus den Fig. 5 und 6 ersichtlich ist, weist die starre Tastenplatte 50 an der ersten Taste 51 an einem distalen Ende einen ersten erhöhten Bereich 51a auf und an der zweiten Taste 52 an einem proximalen Ende der starren Tastenplatte 50 einen zweiten erhöhten Bereich 52a auf. Ausgehend vom ersten erhöhten Bereich 51a verjüngt sich die Tastenplatte in Richtung zur dritten Taste 53. Ebenfalls ausgehend vom zweiten erhöhten Bereich 52a verjüngt sich die Tastenplatte in Richtung zur dritten Taste 53. Die dritte Taste 53 weist in der Mitte ebenfalls einen knopfförmigen dritten erhöhten Bereich 53a auf.

Somit weist jede Taste unterschiedliche geometrische Abmessungen auf, welche einfach durch Überstreichen der Tasten mittels eines Daumens erfühlt werden können. Dadurch ist eine intuitive Bedienung der Tastenanordnung 5 möglich, ohne dass ein Nutzer den Blick auf die Tastenanordnung richten müsste. Dadurch können Operationen, bei denen der Chirurg üblicherweise an einem Bildschirm eine Position des medizinischen Instruments im Inneren des Körpers überwacht, sicher durchgeführt werden. Da die Tastenplatte 50 starr ist, ergibt sich eine besonders gute Rückmeldung für den Nutzer. Insbesondere kann durch Verwendung der starren Tastenplatte 50 immer sicher festgestellt werden, ob eine Taste nun gedrückt ist oder nicht. Dies ist ein besonders großer Vorteil gegenüber anderen möglichen Ausgestaltungen von Tasten mit weichen Bereichen.

Wie weiter aus Fig. 5 ersichtlich ist, sind der erste, zweite und dritte erhöhte Bereich 51a, 52a, 53a von der Mittelachse X-X im unbetätigten Zustand gleich weit entfernt (Abstand 14). Hierdurch können die Höhenunterschiede der ersten, zweiten und dritten Taste 51, 52, 53 auf einfache Weise ertastet werden.

Die Rückstellung der starren Tastenplatte 50 in die in Fig. 5 gezeigte Ausgangsposition erfolgt dann durch Rückstellung der Rückstellelemente 6, 7. Da das erste und zweite Rückstellelement 6, 7 eine Schnappscheibe ist, kann auch eine akustische Mitteilung sowohl bei der Betätigung als auch bei der Rückstellung einer Taste in die Schrappscheiben integriert werden. Dadurch wird die Bedienung des medizinischen Instruments noch komfortabler und sicherer.

Somit kann die Tastanordnung 5 mit drei Tasten 51, 52, 53 und zwei Tastkontakten 55, 56 drei unterschiedliche Betriebsmodi des medizinischen Instruments 1 bereitstellen. Beispielsweise kann die erste Taste einen Linkslauf des inneren Schneidenrohrs bewirken, die zweite Taste einen Rechtslauf des inneren Schneidenrohrs bewirken und die dritte Taste eine oszillierende Hin- und Herbewegung des inneren Schneidenrohrs ermöglichen.

Weiterhin umfasst das Handstück 2 eine Absaugregulierung 54, welche, wie in Fig. 1 gezeigt, in Reihe nach der Tastenanordnung 5 angeordnet ist. Dadurch kann ein Nutzer des medizinischen Instruments üblicherweise mit einer Hand bzw. einem Finger die Tasten der Tastanordnung 5 sowie die Absaugregulierung 54 zur Regulierung der Absaugstärke des medizinischen Instruments betätigen.

Somit kann ein medizinisches Instrument 1 bereitgestellt werden, welches eine Verbindungsanordnung 4 aufweist, die eine sehr einfache Verbindung zwischen Schneidenhalter 3 und Handstück 2 sowie ein sehr einfaches Lösen und Befestigen des Schneidenhalters 3 vom/am Handstück 2 ermöglicht. Durch die über das Gehäuse des Handstücks 2 herausstehenden Drückbereiche 42 der Verbindungsanordnung 4 kann auch auf visuelle Weise ein einfaches Betätigen zum Lösen des Schneidenhalters 3 vom Handstück 4 ermöglicht werden.

Weiterhin kann durch das Vorsehen von zwei Armen 40, welche einander gegenüberliegend am Handstück 2 angeordnet sind, ein unbeabsichtigtes Lösen des Schneidenhalters 3 vom Handstück 2 verhindert werden. Dies ist insbesondere bei einer Verwendung des medizinischen Instruments 1 wichtig, da ein Nutzer üblicherweise das Handstück im Bereich der Tastenanordnung 4 und somit nahe an der Verbindungsanordnung 4 zwischen Handstück 2 und Schneidenhalter 3 hält. Dadurch kann nicht vollständig ausgeschlossen werden, dass der Nutzer unabsichtlich mit einem Finger auf den Drückbereich 42 drückt. Durch das Vorsehen von zwei einander gegenüberliegenden Drückbereichen 42 an dem Handstück 2 ist jedoch selbst in dem unbeabsichtigten Fall, dass der Nutzer aus Versehen auf einen Drückbereich 42 drückt, der Schneidenhalter 3 trotzdem noch sicher am Handstück 2 fixiert. Damit kann während einer Operation, wenn sich der Schneidenhalter 3 innerhalb eines Körperteils eines Patienten befindet, ein unbeabsichtigtes Lösen des Schneidenhalters 3 vom Handstück 2 verhindert werden.

Das Verbinden des Schneidenhalters 3 mit dem Handstück 4 kann durch einfaches axiales Aufschieben des Schneidenhalters 3 auf das Handstück 4 ermöglicht werden, da die beiden U-förmigen Ausnehmungen 45 im Handstück 2 mit ihren Wänden eine Führung der Verbindungsanordnung 4 an den Drückbereichen 42 ermöglichen. Weiterhin kann der Ring 46 mit den integral daran gebildeten Armen 40 auch vom Schneidenhalter 3 gelöst werden und somit Schneidenhalter 3 und Verbindungsanordnung 4 separat gereinigt werden.

Die erfindungsgemäße Tastenanordnung 5 mit starrer Tastenplatte 50, drei Tasten und nur genau zwei Tastkontakten verbessert die Handhabung des medizinischen Instruments weiter in überraschender Weise. Neben einer baulichen Vereinfachung der Tastenanordnung ist auch eine sichere Betätigung der drei Tasten 51, 52, 53 und der Absaugregulierung 54 möglich. Insbesondere sind bei der erfindungsgemäßen Tastenanordnung 5 weniger Kunststoffteile notwendig, da alle Bauteile der Tastenanordnung 5 aus einem Metallmaterial herstellbar sind. Dies hat insbesondere Vorteile bei einer Reinigung mit Flüssigkeiten, da Kunststoffteile nur sehr langsam oder unter zusätzlichem Aufwand trocknen. Dies ist bei der Verwendung von Metallteilen nicht der Fall. Insbesondere in Verbindung mit der Verbindungsanordnung 4 kann somit ein deutlich verbessertes medizinisches Instrument bereitgestellt werden, welches eine deutlich vereinfachte Handhabung und bessere haptische Eigenschaften im Betrieb aufweist.

### Bezugszeichenliste

- 1: medizinisches Instrument
- 2: Handstück
- 3: Schneidenhalter
- 4: Verbindungsanordnung
- 5: Tastenanordnung
- 6: Steuerplatine
- 7: erstes Rückstellelement / erste Schnappscheibe
- 8: zweites Rückstellelement / zweite Schnappscheibe
- 11: erstes Gehäuse für den ersten Tastkontakt
- 12: zweites Gehäuse für den zweiten Tastkontakt
- 13: Halteplatte für erstes und zweites Gehäuse
- 14: Abstand
- 20: Handstückkörper
- 21: Absaugkanal
- 22: Hohlraum im Handstückkörper
- 30: äußeres Hohlrohr
- 31: inneres Schneidenrohr
- 32: Schneiden
- 33: Austrittsöffnung
- 40: Arme
- 41: Rastelement
- 41a: Rampe
- 42: Drückbereich
- 43: elastischer Bereich
- 44: Aufnahme
- 44a: Hinterschneidung
- 45: U-förmige Ausnehmung
- 46: Ring
- 47: Clips-Verbindung
- 50: Tastenplatte
- 51: erste Taste
- 51a: erster erhöhter Bereich
- 52: zweite Taste
- 52a: zweiter erhöhter Bereich
- 53: dritte Taste
- 53a: dritter erhöhter Bereich / knopfartiger Bereich
- 54: Absaugregulierung
- 55: erster Tastkontakt
- 56: zweiter Tastkontakt
- 57: Deckel
- 58: seitlicher Vorsprung
- 59: seitliche Aussparung im Deckel
- A: Betätigung
- B: Wippbewegung
- X-X: Axialrichtung
- Y-Y: Kippachse der Tastenanordnung

## Patentansprüche

1. Medizinisches Instrument für arthroskopische Eingriffe, umfassend:
- ein Handstück (2),
- einen Schneidenhalter (3) mit einem äußeren Hohlrohr (30) und einem inneren hohlen Schneidenrohr (31) mit Schneiden (32) an einem distalen Ende des Innenrohrs, und
- eine Tastenanordnung (5) zur Betätigung des medizinischen Instruments, wobei die Tastenanordnung (5) am Handstück (2) nahe an einem distalen Ende des Handstücks (2) angeordnet ist,
- wobei die Tastenanordnung (5) eine erste Taste (51), eine zweite Taste (52) und eine dritte Taste (53) sowie einen ersten Tastkontakt (55) und einen zweiten Tastkontakt (56) aufweist, wobei die erste Taste (51) eingerichtet ist, den ersten Tastkontakt (55) zu betätigen, die zweite Taste (52) eingerichtet ist, den zweiten Tastkontakt (56) zu betätigen,
**dadurch gekennzeichnet, dass** die dritte Taste (53) dazu eingerichtet ist, gleichzeitig den ersten und zweiten Tastkontakt (55, 56) zu betätigen
- wobei die Tasten (51, 52, 53) an einer gemeinsamen, starren Tastenplatte (50) angeordnet sind
- die starre Tastenplatte (50) lose an der Tastenanordnung (5) angeordnet ist, und lose auf dem ersten und zweiten Tastkontakt (55, 56) aufliegt, und an einer ersten und zweiten axialen Seite an einem Deckel (57) der Tastenanordnung (5) gehalten ist.

2. Instrument nach Anspruch 1,
- wobei die drei Tasten (51, 52, 53) der Tastanordnung (5) in Reihe in Axialrichtung (X-X) derart angeordnet sind, dass die dritte Taste zwischen der ersten und zweiten Taste angeordnet ist und/oder
- wobei die Tastenanordnung (5) an einer Oberseite des Handstücks (2) angeordnet ist.

3. Instrument nach einem der vorhergehenden Ansprüche, wobei die starre Tastenplatte (50) aus einem Metallmaterial, insbesondere aus Edelstahl hergestellt ist.

4. Medizinisches Instrument nach einem der vorhergehenden Ansprüche, wobei die starre Tastenplatte (50) an der ersten Taste (51) an einem distalen Ende einen ersten erhöhten Bereich (51a) aufweist, an der zweiten Taste (52) an einem proximalen Ende der Tastenplatte einen zweiten erhöhten Bereich (52a) aufweist und an der dritten Taste (53) in der Mitte zwischen der ersten und zweiten Taste einen dritten erhöhten Bereich (53a) aufweist.

5. Medizinisches Instrument nach Anspruch 4, wobei ein Abstand (14) des ersten erhöhten Bereichs (51a), des zweiten erhöhten Bereichs (52a) und des dritten erhöhten Bereichs (53a) von einer Mittelachse (X-X) des medizinischen Instruments gleich ist.

6. Medizinisches Instrument nach Anspruch 4 oder 5, wobei sich die erste Taste (51) in Richtung zur dritten Taste (53) verjüngt und wobei sich die zweite Taste (52) in Richtung zur dritten Taste (53) verjüngt.

7. Medizinisches Instrument nach einem der vorhergehenden Ansprüche, wobei die Tastenanordnung (5) ferner eine Steuerungsplatine (6) umfasst, welche in die Tastenanordnung (5) integriert ist.

8. Medizinisches Instrument nach Anspruch 7, ferner umfassend ein erstes Rückstellelement (7) und ein zweites Rückstellelement (8), wobei das erste Rückstellelement (7) zwischen der Steuerplatine (6) und dem ersten Tastkontakt (55) angeordnet ist und das zweite Rückstellelement (8) zwischen der Steuerplatine (6) und dem zweiten Tastkontakt (56) angeordnet ist.

9. Medizinisches Instrument nach Anspruch 8, wobei das erste und zweite Rückstellelement jeweils Schnappscheiben sind, welche bei Verformung ein akustisches Signal abgeben.

10. Medizinisches Instrument nach einem der vorhergehenden Ansprüche, wobei die dritte Taste (53) eine insbesondere zylindrische Erhebung auf der starren Tastenplatte (50) ist und/oder wobei die starre Tastenplatte (50) eine symmetrische Einschnürung im Bereich der dritten Taste (53) aufweist.

## Claims

1. A medical instrument for arthroscopic procedures, comprising:
- a handpiece (2),
- a blade holder (3) with an outer hollow tube (30) and an inner hollow blade tube (31) with blades (32) at a distal end of the inner tube, and
- a button arrangement (5) to operate the medical instrument, wherein the button arrangement (5) is arranged at the handpiece (2) close to a distal end of the handpiece (2),
- wherein the button arrangement (5) comprises a first button (51), a second button (52) and a third button (53) as well as a first button contact (55) and a second button contact (56), wherein the first button (51) is configured to operate the first button contact (55), the second button (52) is configured to operate the second button contact (56),
**characterized in that** the third button (53) is configured to operate the first and second button contacts (55, 56) simultaneously,
- wherein the buttons (51, 52, 53) are arranged at a common, rigid button plate (50),
- the rigid button plate (50) is arranged loosely at the button arrangement (5), and rests loosely on the first and second button contacts (55, 56), and is held at a first and second axial side at a cover (57) of the button arrangement (5).

2. The instrument according to claim 1,
- wherein the three buttons (51, 52, 53) of the button arrangement (5) are arranged in series in axial direction (X-X), such that the third button is arranged between the first and second buttons, and/or
- wherein the button arrangement (5) is arranged at an upper side of the handpiece (2).

3. The instrument according to any of the preceding claims, wherein the rigid button plate (50) is made of a metal material, in particular stainless steel.

4. The medical instrument according to any of the preceding claims, wherein the rigid button plate (50) comprises a first raised portion (51a) at the first button (51) at a distal end, a second raised portion (52a) at the second button (52) at a proximal end of the button plate, and a third raised portion (53a) at the third button (53) in the middle between the first and second buttons.

5. The medical instrument according to claim 4, wherein a distance (14) of the first raised portion (51a), the second raised portion (52a) and the third raised portion (53a) from a center axis (X-X) of the medical instrument is the same.

6. The medical instrument according to claim 4 or 5, wherein the first button (51) tapers towards the third button (53) and wherein the second button (52) tapers towards the third button (53).

7. The medical instrument according to any of the preceding claims, wherein the button arrangement (5) further comprises a control board (6), which is integrated into the button arrangement (5).

8. The medical instrument according to claim 7, further comprising a first resetting element (7) and a second resetting element (8), wherein the first resetting element (7) is arranged between the control board (6) and the first key contact (55) and the second resetting element (8) is arranged between the control board (6) and the second key contact (56).

9. The medical instrument according to claim 8, wherein the first and second resetting elements are each snap discs, which emit an acoustic signal when deformed.

10. The medical instrument according to any of the preceding claims, wherein the third button (53) is a particularly cylindrical protrusion on the rigid button plate (50) and/or wherein the rigid button plate (50) comprises a symmetrical narrowing in the portion of the third button (53).

## Revendications

1. Instrument médical pour des interventions arthroscopiques, comprenant :
- une pièce à main (2),
- un support de coupe (3) avec un tube creux externe (30) et un tube de coupe creux interne (31) avec des bords tranchants (32) sur une extrémité distale du tube interne, et
- un ensemble de touches (5) pour actionner l'instrument médical, dans lequel l'ensemble de touches (5) est disposé sur la pièce à main (2) à proximité d'une extrémité distale de la pièce à main (2),
- dans lequel l'ensemble de boutons (5) présente un premier bouton (51), un deuxième bouton (52) et un troisième bouton (53) ainsi qu'un premier contact de bouton (55) et un deuxième contact de bouton (56), dans lequel le premier bouton (51) est mis au point pour actionner le premier contact de bouton (55), le deuxième bouton (52) est mis au point pour actionner le deuxième contact de bouton (56)
**caractérisé en ce que** le troisième bouton (53) est mis au point pour actionner simultanément le premier et deuxième contact de bouton (55, 56),
- dans lequel les boutons (51, 52, 53) sont disposés sur une plaque de boutons rigide (50) commune,
- la plaque de boutons rigide (50) est disposée de manière lâche sur l'ensemble de boutons (5), et repose de manière lâche sur le premier et le deuxième contact de bouton (55, 56), et est maintenue sur un couvercle (57) de l'ensemble de boutons (5) sur un premier et un deuxième côté axial.

2. Instrument selon la revendication 1,
- dans lequel les trois boutons (51, 52, 53) de l'ensemble de boutons (5) sont disposés en série dans la direction axiale (X-X) de telle manière que le troisième bouton est disposé entre le premier et le deuxième bouton, et/ou
- dans lequel l'ensemble de boutons (5) est disposé sur un côté supérieur de la pièce à main (2).

3. Instrument selon l'une quelconque des revendications précédentes, dans lequel la plaque de boutons rigide (50) est fabriquée à partir d'un matériau métallique, en particulier en acier inoxydable.

4. Instrument médical selon l'une quelconque des revendications précédentes, dans lequel la plaque de boutons rigide (50) présente, sur le premier bouton (51), sur une extrémité distale, une première zone surélevée (51a), sur le deuxième bouton (52), sur une extrémité proximale de la plaque de boutons, une deuxième zone surélevée (52a) et présente, sur le troisième bouton (53), au centre entre le premier et le deuxième bouton, une troisième zone surélevée (53a).

5. Instrument médical selon la revendication 4, dans lequel une distance (14) de la première zone surélevée (51a), de la deuxième zone surélevée (52a) et de la troisième zone surélevée (53a) par rapport à un axe central (X-X) de l'instrument médical est identique.

6. Instrument médical selon la revendication 4 ou 5, dans lequel le premier bouton (51) se rétrécit en direction du troisième bouton (53) et dans lequel le deuxième bouton (52) se rétrécit en direction du troisième bouton (53).

7. Instrument médical selon l'une quelconque des revendications précédentes, dans lequel l'ensemble de boutons (5) comprend en outre une platine de commande (6), laquelle est intégrée dans l'ensemble de boutons (5).

8. Instrument médical selon la revendication 7, comprenant en outre un premier élément de rappel (7) et un deuxième élément de rappel (8), dans lequel le premier élément de rappel (7) est disposé entre la platine de commande (6) et le premier contact de bouton (55) et le deuxième élément de rappel (8) est disposé entre la platine de commande (6) et le deuxième contact de bouton (56).

9. Instrument médical selon la revendication 8, dans lequel le premier et le deuxième élément de rappel sont respectivement des rondelles à déclic, lesquelles émettent un signal acoustique en cas de déformation.

10. Instrument médical selon l'une quelconque des revendications précédentes, dans lequel le troisième bouton (53) est une partie surélevée en particulier cylindrique sur la plaque de boutons rigide (50) et/ou dans lequel la plaque de boutons rigide (50) présente un resserrement symétrique dans la zone du troisième bouton (53).
